# EUROPEAN PATENT SPECIFICATION

(11) **EP 2 311 514 B1**
(45) Date of publication and mention of the grant of the patent: **09.05.2018**
(21) Application number: 09800568.9
(22) Date of filing: 22.07.2009
(51) Int. Cl.: A61M 5/38, A61M 5/44

(54) **MEDICAL HEATING DEVICE**
MEDIZINISCHE HEIZVORRICHTUNG
DISPOSITIF DE CHAUFFAGE MÉDICAL

(30) Priority: 22.07.2008 KR 20080071344
(43) Date of publication of application: 20.04.2011
(73) Proprietor: Wooyoung Meditech Co., Ltd., Seocho-gu, Seoul 137-903 (KR)
(72) Inventor: Park Jae-Sang, Seoul 137-937 (KR)
(74) Representative: Neobard, William John
(86) International application number: PCT/KR2009/004078
(87) International publication number: WO 2010/011087

(56) References cited:
- WO-A1-2005/027578
- JP-A- 2004 105 599
- KR-B1- 100 350 576
- KR-B1- 100 553 129
- US-A- 5 381 510
- US-A- 5 420 962
- US-B1- 7 236 694
- SUNG WON JUNG ET AL.: 'Performance Characteristics of High Efficiency Fluid and Blood Warmer using Print Circuit Board Heater at Various Flow Rates' JOURNAL OF THE KOREAN SOCIETY OF ANESTHESIOLOGISTS vol. 51, 05 November 2006, pages 598 - 605, XP008142743

## Description

### [Technical Field]

The present invention relates to a warming device for a medical treatment.

### [Background Art]

Conventionally, when injecting a liquid or blood to human body, a device required for injecting the liquid or blood by getting the liquid or blood to be warm up at similar temperature to a body temperature.

The liquid or blood is maintained at a lower temperature in order to prevent them from rotten or prevent an occurrence of harmful materials therein. In the event that the liquid or blood was injected to the human body, an energy for the patient's metabolism is required to maintain the temperature of the injected liquid or blood equal to that of the patient's body. Further, in the event that the liquid or blood of lower temperature is injected to the patient, it may drop the temperature of the patient's body and cause the patient to have a heart attack, even death. In particular, in case of a patient under a general anesthetic where the patent's temperature cannot be controlled, it makes the patent feel the cold severely after the operation and stimulates a cooling point in the tissue to which the liquid and blood is injected to thereby feel a cooling pain

Accordingly, prior to injecting a liquid or blood to the patient's body, it is necessary to warm the liquid or blood at similar temperature to the patient's body temperature.

Also, in the event that the liquid or blood is getting warm up, a gas may be generated. If the generated gas is not properly removed, it may block the smooth circulation of the blood, and particularly when a capillary is blocked, it may cause a necrosis of cell resulting in occurrence of side effects such as aeroembolism.

WO 2005/027578 discloses a heating apparatus having a PCB-type heater capable of allowing the temperature of Ringer's solution or blood which is introduced into a blood vessel, when transfusing a blood thereto, to be equal to that of the human body to thereby provide an accurate resistance value. The heating apparatus of WO 2005/027578 comprises a body including a first connecting portion having a tube which is connected to the first connection portion and receives a fluid supplied from an instillation room, a path having the shape of a spiral screw thread to enable the fluid to flow and a second connecting portion for supplying the fluid flowing through the path to an injection syringe; an inner cover inserted into the body and fixedly attached thereto in a prescribed adhesive manner for preventing the fluid from flowing out; a middle cover for closely fixing the body and the inner cover against each other; a PCB-type heater inserted into the inside of the top and bottom surfaces of the middle cover for heating the fluid flowing through the path so that the fluid is maintained at a prescribed temperature; a bottom case having a box portion to receive the body having the heater and the middle cover coupled thereto; and a upper case coupled to the bottom case.

### [Summary of Invention]

### [Technical Problem]

An embodiment of the present invention provides a warming device for medical treatment.

An embodiment of the present invention provides a warming device for medical treatment capable of effectively warming a liquid or blood.

An embodiment of the present invention provides a warming device for medical treatment capable of effectively removing an air generated in a liquid or blood.

An embodiment of the present invention provides a warming device for medical treatment for allowing a liquid or blood to be smoothly moved.

### [Solution to Problem]

According to the invention there is provided a warming device comprising a heater having a first side and a second side; a first connection portion into which fluid flows from an external supplier; a second connection portion from which warmed fluid is discharged; a plurality of barrier members that are disposed on each of the sides of the heater and the warming device further comprises an air filter disposed in the fluid path to remove air from the fluid, wherein the air filter is disposed on an internal side of any one of the cover members; wherein the warming device is configured in such a manner that the pair of cover members are each disposed on opposing sides of the heater, the pair of cover members combines the heater and the barrier members together, and a fluid path surrounding the heater several times is formed between the heater and the barrier members, so that the fluid flowing through the fluid path from the first connection portion is warmed and the warmed fluid is supplied to the second connection portion. A warming device disclosed herein comprises a heater; a barrier member and a cover member which are disposed at a front side and a rear side of the heater, respectively and forming a fluid path that allows the fluid to be moved in a manner of surrounding the heater; an air filter portion disposed on the fluid path and configured to remove an air; a first connecting portion for allowing the fluid to inflow into the fluid path; and a second connection portion for allowing the fluid to be discharged from the fluid path.

A warming device disclosed herein comprises a heater formed of a PCB substrate having a resistance pattern provided at front and rear sides; a barrier member and a cover member which are disposed a front side and a rear side of the heater, respectively and forming a fluid path that allows the fluid to be moved in a manner of surrounding the heater; a filter disposed on the fluid path between the heater and the cover member; a void formed on a cover member at the position corresponding to the filter; a first connecting portion for allowing the fluid to inflow into the fluid path; and a second connection portion for allowing the fluid to be discharged from the fluid path.

### [Advantageous Effects of Invention]

An embodiment of the present invention can provide a warming device for medical treatment.

An embodiment of the present invention can provide a warming device for medical treatment capable of effectively warming up a liquid or blood.

An embodiment of the present invention provides a warming device for medical treatment capable of effectively removing an air generated in a liquid or blood.

An embodiment of the present invention can provide a warming device for medical treatment for allowing a liquid or blood to be smoothly moved.

### [Brief Description of Drawings]

Figure 1 is a perspective view of a warming device for medical treatment according to an embodiment of the present invention.
Figure 2 is a view showing the assembled state of the warming device for medical treatment according to an embodiment of the present invention.
Figure 3 is a front view of the heater.
Figure 4 is a rear view of the heater.
Figure 5 is a view showing a state that a first cover and a heater are combined in the warming device according to an embodiment of the present invention.
Figure 6 is a view showing a state that a first cover, a heater and a second cover are combined in the warming device according to an embodiment of the present invention.
Figure 7 is a view showing that a connector formed within a lower case, voltage applying electrodes of the heater and a sensing electrode are connected in the warming device according to an embodiment of the present invention.
Figure 8 is a cross-sectional view of a state that a first cover, a heater, a second cover and a third cover are coupled together in the warming device according to an embodiment of the invention.
Figures 9 to 12 are views illustrating a warming device according to another embodiment of the present invention.
Figure 13 is a view illustrating a warming device according to a further another embodiment of the present invention.
Figure 14 is a view illustrating a warming device according to a still further another embodiment of the present invention.
Figures 15 and 16 are views showing electrical connecting relations of voltage connectors.

### [Description of Embodiments]

In describing embodiments according to the present invention, in the event that each layer (film) area, pattern or structure are described to be formed "on" or "under" a substrate, each layer (film), area, pad or patterns, the terms "on" and "under" is intended to cover the meaning of "directly "on" and "under" or "indirectly "on" and "under" through other layer. Also, the criterion of on/under each layer is described on the basis of the drawings.

In the drawings, the thickness or size of each layer is illustrated exaggeratedly, abbreviately or schematically for the convenience and clearness of the explanation. Also, the size of each constituent element does not fully reflect its actual size.

Now, a warming device for medical treatment according to embodiments of the present invention will be described in detail with reference to the accompanying drawings.

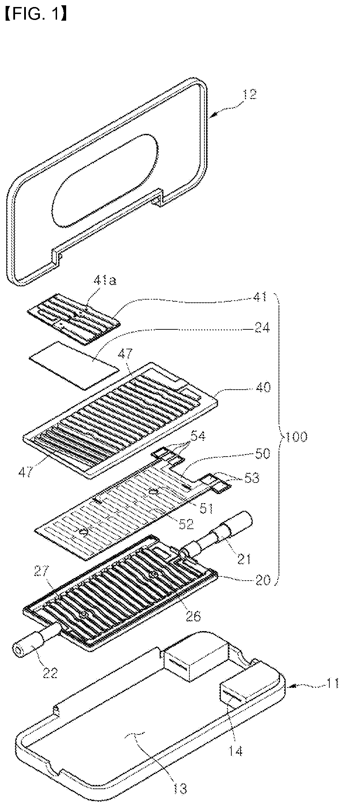

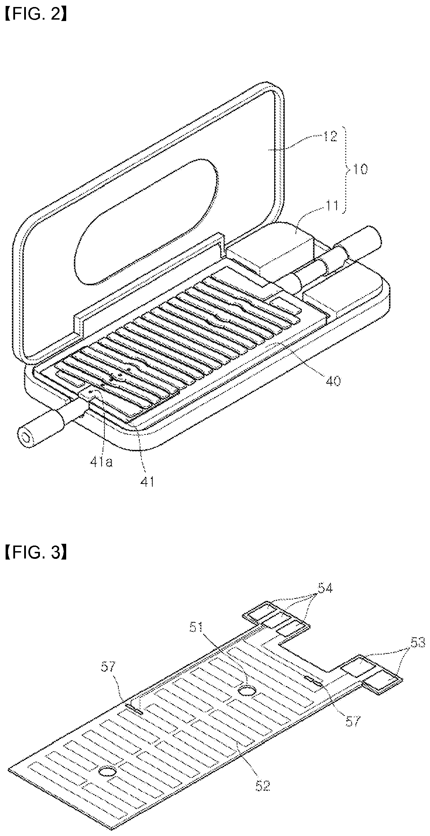

Figure 1 is a perspective view of a warming device for medical treatment according to an embodiment of the present invention, and Figure 2 is a view showing an assembly of a warming device for medical treatment according to an embodiment of the present invention.

Referring to Figures 1 and 2, the warming device for medical treatment according to an embodiment of the present invention is provided with a case in which a lower case (11) and a upper case (12) are coupled each other. A warming device (100) is disposed on a mounting portion(13) between the lower case (11) and the upper case (12).

The warming device for medical treatment according to an embodiment of the present invention provides a warming function for warming such fluid as blood and liquid and an air filtering function for removing an air in the fluid. In order to provide such functions, the warming device (100) according to the embodiment includes a first cover (20), a heater (50), a second cover (40), a filter (24) and a third cover (41).

The first cover (20) and the second cover (40) are coupled to each other with the heater sandwiched therebetween, and the second cover (40) and the third cover (41) are coupled to each other with the filter (24) sandwiched therebetween.

A plurality of first barriers (27) are formed on a side opposite to the heater (50) of the first cover (20) and a plurality of second barriers (47) are formed on a side opposite to the heater (50) of the second cover(20). The first cover (20) and the first barrier (27) may be formed by the integral injection molding, and similarly the second cover (40) and the second barrier (47) may be formed by means of the integral injection molding.

The second cover (40) is provided with the second barrier (47) so that the second barrier (47) can form a fluid path together with the first barrier (27) of the first cover (20) corresponding thereto in which a partial area of the second cover (40) is provided with only the second barrier (47) without being provided with a partial area of the second cover supporting the second barrier (47) in a upper direction. The second barrier (47) is, at both ends thereof, supported by the second cover (40).

On a partially removed portion of the second cover (40), the filter (24) is disposed and the third cover (41) is also disposed for supporting the filter (24) and forming the fluid path. The third cover (41) is provided with a plurality of voids (41a) for outwardly discharging an air generated from respective fluid path partitioned by the second barrier (47).

The first barrier (27) is formed to be sloped in a first direction and the second barrier (47) is formed to be sloped in a second direction to thereby form a fluid path together with the heater (50).

The fluid path formed of a first cover (20) containing the first barrier (27), a second cover (40) containing the second barrier (47), third cover (41) and a heater (50) is configured to surround the heater in the form of screw thread so that a heat generated from the heater (50) can be sufficiently delivered to the fluid flowing through the fluid path.

Because the width of the heater (50) is narrower than that of the formed first and second barriers (27, 47), a front fluid path and a rear fluid path which are partitioned by the heater (50) are connected to each other at both sides of the heater (50).

The first cover (20) includes a first connection portion (21) into which a fluid flows and a second connection portion (22) from which a fluid is discharged. Therefore, in the event that a first cover (20) including the first barrier (27), a heater (50), a second cover (40) including the second barrier (47) and a third cover (41) are combined together, the fluid inflows into the first connection portion (21) and is discharged through the second connection portion (22) via the fluid path formed of the first cover (20) including the first barrier (27), the second cover (40) including the second barrier (47), the third cover (30) and the heater (50).

Because the warming device (100) is, at its upper side, disposed with the first connection portion (21) and is, at its lower side, disposed with the second connection portion (22), the fluid inflowing through the first connection portion (21) is discharged through the second connection portion along with the fluid path.

For example, the fluid inflowing through the first connection portion (21) flows through a rear fluid path formed by the rear side of the heater (50), the first cover(20) and the first barrier (27) of the first cover (20) to a first sloped direction (that is, a right downward direction) of the first barrier (27) and through a front fluid path formed, at the end of the rear side fluid path, by the front side of the heater (50), the second cover (40) and the second barrier(47) of the second cover (40) to a second sloped direction (that is, left downward direction) of the second barrier (47).

Further, the fluid flows through the rear fluid path formed, at a portion adjacent to the second connection portion (21), by the rear side of the heater (50), the first cover (20) and the first barrier (27) of the first cover (20) and flows through the front fluid path formed, at the end of the rear fluid path, by the front side of the heater(50), the second barrier (47) of the second cover(40) and the third cover (41) to the second connection portion (21).

As shown, the filter (24) is disposed adjacent to the first connection portion rather than the first connection portion (21). Because the fluid includes more airs at the warmed temperature than at a low temperature, it is more effective to dispose the filter (24) adjacent to the second connection portion (22) in providing air filtering effects.

As such, the fluid path which is formed by the first cover (20) including the first barrier (27), the heater (50), the second cover (40) including the second barrier (47) and the third cover (41) is configured to surround the filter (50) in the form of screw thread. Accordingly, the fluid inflowing through the first connection portion (21) can be sufficiently warmed by the heater (50) and the pressure of the fluid flowing through the fluid path becomes more powerful than that outside of the second cover (40) and the third cover (41) and thus the air in the fluid is discharged to the outside through the filter (24). Therefore, the air is removed from the fluid and the fluid is discharged to the outside through the second connection portion (22).

The filter (24) is disposed at the upper side of the front fluid path horizontally with the flowing of the fluid. For example, the filter (24) may be formed of a hydrophobic non-woven fabric having less affinity with the fluid and having fine voids formed therein. The fine void has its size in the extent of preventing the fluid from passing through it. The filter (24) can be attached to the third cover (41) using an ultrasonic wave or an adhesive. The third cover (41) is formed with a plurality of voids (41a) to allow an air collected by the filter (24) to be discharged to the outside.

Although not shown, the plurality of voids (41a), the filter (24) and the third cover (41) which function as an air filter may be formed on the front fluid path as well as the rear fluid path.

In the warming device (100) according to the embodiment, the filter (24) is disposed at the position opposite to the heater (50) thereby performing both warming function and air filtering function at the same time. Therefore, it is possible to promptly remove an air from the fluid warmed in the warming device (100) without using a drip chamber for removing the air. Further, the warming device can be disposed near the patient maximally to thereby allow injection of the warmed fluid to the patient before the temperature of the warmed fluid falls low. Also, it is not necessary to use the drip chamber, resulting in an advantage that there is no need to make the first and second connection portions (21, 22) stand up and down vertically.

In the warming device (100) according an embodiment, the first connection portion (21), the first cover (20) and the second connection portion (22) can be formed integrally by an injection molding. Further, in the warming device (100) according another embodiment, the second cover (40) and the second barrier (47) can be formed integrally by an injection molding. The heater (50) may be a PCB (Printed Circuit Board) substrate having resistive patterns (52) formed thereon.

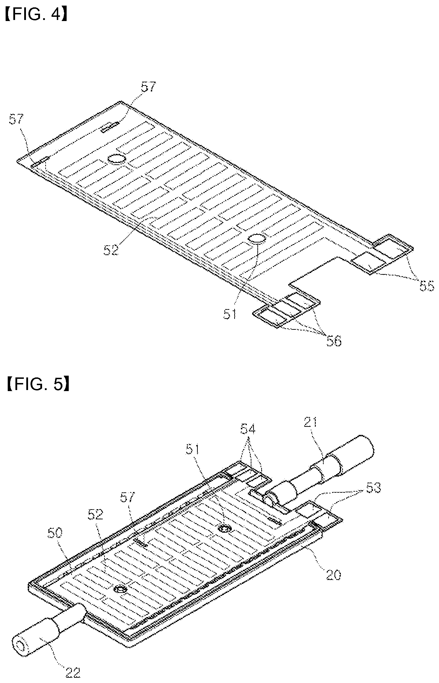

Figure 3 is a front view of the heater and Figure 4 is a rear view of the heater. Referring to Figures 3 and 4 together, the heater (50) has resistive patterns (52) formed on the front and rear sides thereof and thus a heat is emitted in the resistive patterns (52) by a voltage applied through the voltage applying electrodes (53, 55). Also, the heater (50) has temperature sensors (57) formed at the front and rear sides thereof, and thus the temperature sensors (57) are sensing the temperature of the fluid and the sensed signals are sent through sensing electrodes (54, 56) to the outside.

In the warming device (100) according to the embodiment, in order to effectively perform warming, resistive patterns (52) are formed at the front and rear sides of the heater (50), and the resistive pattern (52) at the front side and the resistive pattern (52) at the rear side are electrically inter-divided because these resistive patterns (25) are manufactured without having a process for electrically inter-connecting them. That is, two voltage applying electrodes (53) formed on the front side of the heater (50) are applied with positive and negative voltages, respectively and similarly, the two voltage applying electrodes (55) formed on the rear side of the heater (50) are applied with positive and negative voltages.

Meanwhile, as described below, it is possible to supply a voltage through a voltage connector (14) and connect the resistive patterns (52) formed at the front and rear sides of the heater (50) in serial or parallel. For example, in case that the resistive patterns (52) formed at the front and rear sides of the heater (50) are not electrically divided, it needs a process for electrically connecting the resistive pattern (52) disposed at the front side of the heater (50) and the resistive pattern (52) disposed at the rear side of the heater (50). To do this, when forming on the heater (50) a via hole in which a conductive material is buried, and electrically connecting the resistive pattern (52) disposed at the front side of the heater (50) and the resistive pattern (52) disposed at the rear side of the heater (50), such conductive material may be attached to the resistive pattern (52) disposed at the front side of the heater (50) and the resistive pattern (52) disposed at the rear side are attached with such conductive material in the course of burying the conductive material into the via hole and resultantly it is not difficult to manufacture resistive patters (52) having exact resistive values.

Also, because the thickness of the conductive material buried in the via groove must be thin than that of the resistive pattern (52), under application of voltage thereto, much heat may be generated to thereby cause a short, and further costs may incur in the course of forming the via groove resulting in the increased production costs of the heater (50).

Accordingly, the resistive pattern (52) disposed on the front side of the heater (50) and the resistive pattern (52) disposed on the rear side thereof are made to be inter-divided electrically, and each resistive pattern (52) is made so as to have voltage applying electrode (53, 55) to which positive and negative voltages are applied.

In similar, the temperature sensor (57) is also formed on the front and rear sides of the heater (50) respectively to be divided electrically and is electrically connected to the sensing electrodes (54, 56) which are formed on the front and rear sides of the heater (50) respectively.

Also, the heater (50) may be provided with a hole (51) and the first cover (20) may be provided with a protrusion (26). The protrusion (26) is inserted into the hole (51) so that the heater (50) can be disposed on an exact position on the first cover (20).

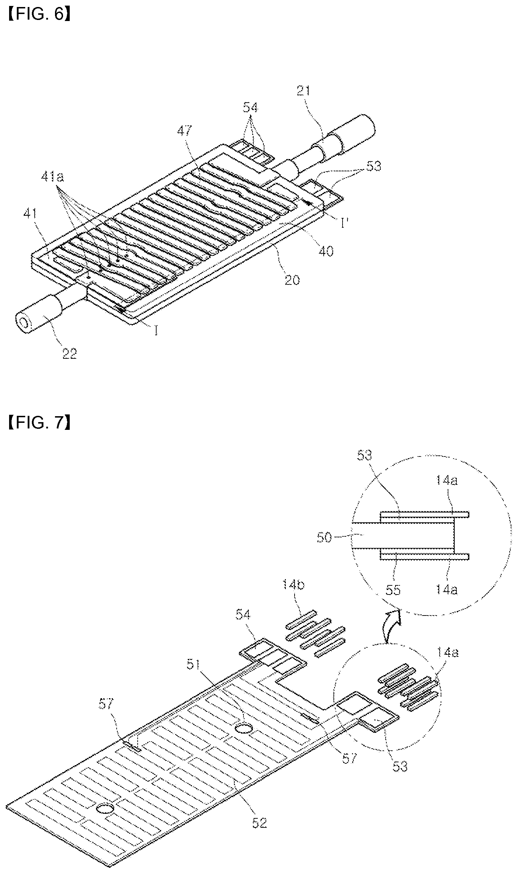

Figure 5 is a view showing a state that a first cover and a heater are combined in the warming device according to the embodiment of the present invention, Figure 6 is a view showing a state that a first cover, a heater and a second cover are combined in the warming device according to the embodiment of the present invention, and Figure 7 is a view showing that a connector formed within a lower case, voltage applying electrodes of the heater and a sensing electrode are connected in the warming device according to the embodiment of the present invention.

Referring to Figures 5 to 7, the heater (50) is disposed to be inserted between the second cover (40) and the first cover (20), and the voltage applying electrodes (53, 55) and sensing electrodes (54, 56) of the heater (50) are protruded outwardly.

The voltage applying electrodes (53, 55) and sensing electrodes (54, 56) of the heater (50) which are protruded outwardly are inserted into the electrode inserting hole (14) of the lower case (11) shown in Figure 1 and are electrically connected to the voltage connector (14a) and the sensor connector (14b) disposed within the lower case(11) as shown in Figure 2.

Figure 7 shows the voltage connector (14a) and the sensor connector (14b) disposed within the lower case (11). Since the voltage connecter (14a) and the sensor connector(14b) should electrically connect the resistive pattern (52) and the temperature sensor formed on the front and rear side of the heater (50) and supply a voltage from the outside, it is preferable to make a double connecter structure in which connectors are disposed on the upper and lower of the heater (50).

The voltage connecter (14a) and the sensor connector (14b) are positioned to correspond to the voltage applying electrodes (53, 55) and the sensing electrodes (54, 56) formed on the front side and the rear side of the heater (50), respectively.

Accordingly, in the event that the voltage applying electrodes (53, 55) and the sensing electrodes (54, 56) are inserted into the electrode inserting hole (14), the voltage applying electrodes (53, 55) and the sensing electrodes (54, 56) formed on the front side and the rear side of the heater (50), respectively are connected simultaneously to the voltage source and the sensor connectors (14a, 14b).

Although not shown, the lower case (11) may be provided with a driving chip for driving the heater (50), a sensing signal processing portion for processing signal obtained by the temperature sensor (57), a display portion for an user, an input button for user's operation, etc which are electrically connected to the voltage source and the sensor connectors (14a,14b).

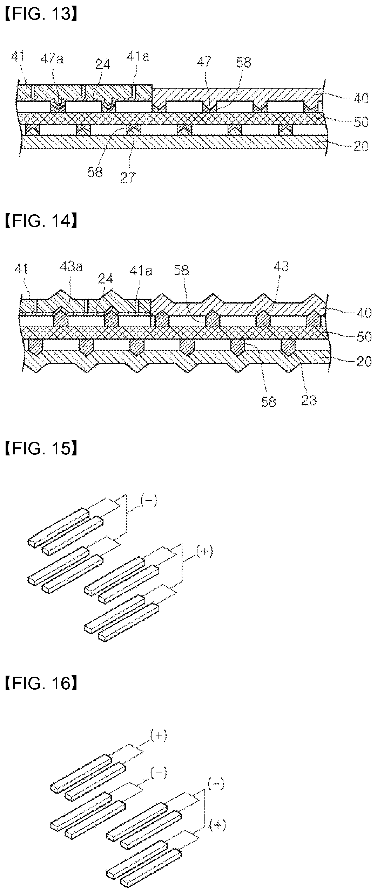

Meanwhile, Figures 15 and 16 illustrate the electric connection relation of the connector for a voltage source.

Figure 15 shows the connection in parallel of the resistive patterns (52) formed on the frond and rear sides of the heater (50), and Figure 16 shows the connection in serial of the resistive patterns (52) formed on the frond and rear sides of the heater (50).

Because the resistive patterns (52) formed on the front side of the heater (50) is electrically divided from the resistive patterns (52) formed on the rear side of the heater (50), the resistive patterns (52) formed on the frond and rear sides of the heater (50) can be electrically connected each other using the voltage connector (14a).

Accordingly, the voltage applying electrodes (53, 54) formed on the frond and rear sides of the heater (50) can be electrically connected each other upon application of voltage. Of course, because the resistance values of the resistive patterns (52) become different depending on the serial and parallel connection schemes, when setting a desired heat amount (that is, desired resistance value), both the connection scheme of the resistive patterns (52) of the front and rear sides of the heater (50) and each resistance value of the resistive patterns (52) formed on the upper and rear sides are considered.

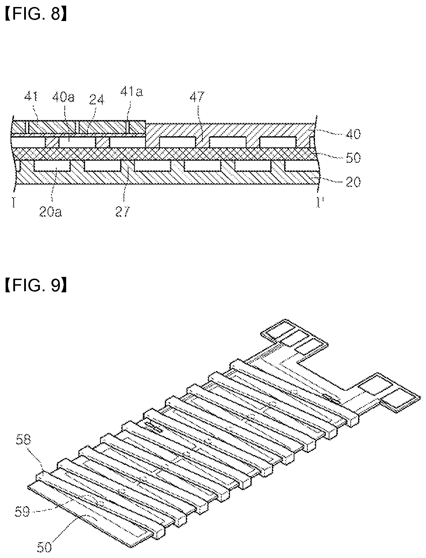

Figure 8 is a cross-sectional view of a state that a first cover, a heater, a second cover and a third cover are coupled together in the warming device according to an embodiment of the invention. Referring to Figure 8, as described above, the first cover (20), the first barrier (27) of the first cover (20) and the rear side of the heater (50) forms the rear fluid path (20a), and the second cover (40), the second barrier (47) of the second cover (40) and the front side of the heater (50) forms the front fluid path (40a).

The rear fluid path (20a) and the front fluid path (40a) cross over at the center portion of the warming device (100) with having the heater (50) sandwiched therebetween according to the directions of the first barrier (27) and the second barrier (47).

Figure 8 is a cross-sectional view of the warming device at the position (I-I' in Figure 6) offset one side from the center portion thereof. Therefore, the first barrier (27) and the second barrier (47) are disposed intercross-wisely.

Meanwhile, when the fluid is passing through the front fluid path on which the filter (24) is formed, an air in the fluid is collected by the filter (24) and then discharged to the outside through the void (41a).

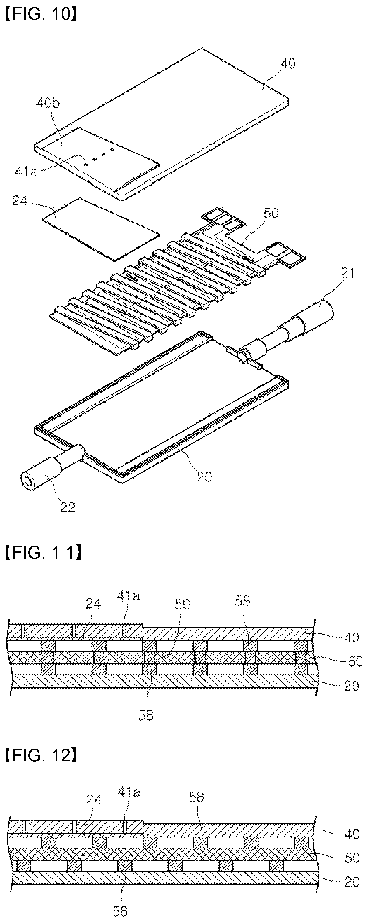

Figures 9 to 12 illustrate a warming device according to another embodiment of the present invention. Figure 9 shows a heater (50) provided with a barrier (58) configured to surround the heater in the form of screw thread, Figure 10 shows an exploded, perspective view of a first cover (20), a heater (50), a filter (24) and a second cover, Figure 11 shows a sectional view of the center portion of the warming device in the state that a first cover (20), a heater (50), a filter (24) and a second cover (40) are coupled, and Figure 12 shows a sectional view of the warming device at the position offset one side from the center portion of the warming device in the state that a first cover (20), a heater (50), a filter (24) and a second cover (40) are coupled.

Referring to Figures 9 to 12, a heater (20) is provided directly with a barrier (58) in contrast with the embodiment described above. The barrier (58) can be attached to the heater (50) by an insert-injection molding together with the heater (50).

The front and rear sides of the heater (50) may be applied with a coating material (not shown) so that when the barrier (58) is insert-injected, the coating material is dissolved to thereby prevent an occurrence of a fine gap between the barrier (580 and the heater. In this case, the coating material can be selected as a material having a melting point lower than the material forming the barrier (58).

According to an embodiment, the center portion of the heater (50) may be provided with a connecting hole (59) at predetermined distance. The connection hole (59) allows the barrier (58) formed on the front and rear sides of the heater (50) to be coupled more securely to the heater (50) by, when the barrier (58) is insert-injected into the hole, permitting the material which forms the barrier (58) to be injected into the connection hole (59).

The barrier (58) is attached to the heater (50) by an insert-injection molding together with the heater (50), but is not attached to the first cover (20) and the second cover (40).

Meanwhile, the second cover (40) is provided with a filter attaching portion (40b) so that the filter (24) can be attached thereto. The filter attaching portion (40b) may be formed by making the thickness of the second cover (40) thinner than other portion or making it to be protruded in the upper direction compared to other portion. Also, in the same manner as described with reference to Figure 8, the filter attaching portion is provided with a void (41a) at the position corresponding to the fluid path and thus an air collected by the filter (24) is discharged to the outside.

Figure 13 illustrates a warming device according to a further another embodiment of the present invention. Referring to Figure 13, the front and rear sides of the heater (50) is provided with a barrier (58) as shown in Figure 9 and the first cover (20) and the second cover (40) are also provided with the first barrier (27) and the second barrier (47), respectively. Further, a third cover (41) having a third barrier (47a) formed thereon may be further provided and the third cover (41) may be formed integral with the second cover (40) as shown in Figure 11.

The barrier (58) formed on the heater (50) is coupled to the heater (50) by an insert-injection, and the first barrier (27), the second barrier (47) and the third barrier (47a) are formed by being insert-injected together when the first cover (20), the second cover (40) and the third cover (41) are insert-injected.

The end of the barrier (58) has a concave shape, and the ends of the first, second and third barriers (27, 47, 47a) have a protuberant shape. Therefore, the ends of the first, second and third barriers (27, 47, 47a) can correspond to the barrier (58) so that the fluid can be smoothly moved along the given fluid path.

Meanwhile, a filter (24) is formed between the heater (50) including the barrier (58) and the third cover (41) including the third barrier (47a) and a void (41a) is formed on the third cover (41) so that an air in the fluid can be removed as described above.

In contrast to the above description that the end of the barrier (58) has a concave shape, and the ends of the first, second and third barriers (27,47,47a) have a protuberant shape, the end of the barrier (58) may have a protuberant shape and the ends of the first, second and third barriers (27, 47, 47a) have a concave shape.

Figure 14 shows a warming device according to a still further another embodiment of the present invention. Referring to Figure 14, the front and rear sides of the heater (50) is provided with a barrier (58) as shown in Figure 9, and holes (23, 43) are formed at the position corresponding to the barrier (58) of the first cover (20) and the second cover (40).

Further, a third cover (41) having a hole (43a) formed thereon may be further provided and the third cover (41) may be formed integral with the second cover (40) as shown in Figure 11. The barrier (58) formed on the heater (50) is coupled to the heater (50) by an insert-injection, and the holes (20a, 40a, 43a) of the first cover (20), the second cover (40) and the third cover (41) are formed when the first cover (20), the second cover (40) and the third cover (41) are insert-injected.

The end of the barrier (58) has a protuberant shape and a part thereof is inserted into the holes (20a, 40a, 43a) of the first cover (20), the second cover (40) and the third cover (41). Therefore, the holes (20a, 40a, 43a) of the first cover (20), the second cover (40) and the third cover (41) can correspond to the barrier (58) so that the fluid can be smoothly moved along the given fluid path.

Meanwhile, a filter (24) is formed between the heater (50) including the barrier (58) and the third cover (41) including the hole (43a) and a void (41a) is formed on the third cover (41) so that an air in the fluid can be removed as described above.

### [Industrial Applicability]

The particular embodiments disclosed above are illustrative only, as the invention may be modified and practiced in different but equivalent manners apparent to those skilled in the art having the benefit of the teachings herein. Furthermore, no limitations are intended to the details of construction or design herein shown, other than as described in the claims below. It is therefore evident that the particular embodiments disclosed above may be altered or modified and all such variations are considered within the scope of the invention. Accordingly, the protection sought herein is as set forth in the claims below.

### [Reference Signs List]

| | |
|---|---|
| 11: lower case | 12: upper case |
| 13: mounting portion | 20: first cover |
| 20a: rear fluid path | 21: first connection portion |
| 22: second connection portion | 24: filter |
| 27: first barrier | 40: second cover |
| 40a: front fluid path | 41: third cover |
| 41a: voids | 47: second barriers |
| 50: heater | 58: barrier |
| 59: connection hole | 100: warming device |

## Claims

1. A warming device (100) comprising:
a heater (50) having a first side and a second side;
a first connection portion (22) into which fluid flows from an external supplier;
a second connection portion (21) from which warmed fluid is discharged;
a plurality of barrier members (27); and
a pair of cover members (20, 40),
wherein the warming device (50) is configured in such a manner that the pair of cover members (20, 40) are each disposed on opposing sides of the heater (50), the pair of cover members (20, 40) combines the heater (50) and the barrier members (27) together, and a fluid path surrounding the heater (50) several times is formed between the heater (50) and the barrier members (27), so that the fluid flowing through the fluid path from the first connection (22) portion is warmed and the warmed fluid is supplied to the second connection portion (21), **characterized in that**: the plurality of barrier members are disposed on each of the sides of the heater, and the warming device (100) further comprises an air filter (24) disposed in the fluid path to remove air from the fluid, wherein the air filter (24) is disposed on an internal side of any one of the cover members (20,40).

2. The warming device (100) according to claim 1, wherein the heater (50) is a PCB type heater formed on an insulating substrate, and the PCB type heater comprises a resistive pattern formed on both sides of the insulating substrate.

3. The warming device (100) according to claim 1, wherein the barrier members (27) are formed on an inner side of each of the cover members (20, 40).

4. The warming device (100) according to claim 3, wherein the cover members (20, 40) and the barrier members (27) each are formed in a single body by an injection molding, respectively, and/or wherein a barrier member of the barrier members (27) on a first cover member (20) of the cover members (20, 40) is inclined in a first direction and a barrier member of the barrier members (27) on a second cover member (40) of the cover members (20, 40) is inclined in a second direction so that the fluid path may be formed in a screw thread shape.

5. The warming device (100) according to claim 1,
wherein the air filter (24) is disposed between an internal side of any one of the cover members (20, 40) and the barrier members (27), or
wherein the air filter (24) is formed of hydrophobic membrane to prevent the fluid from passing through the fluid path or
wherein a part of any one of the cover members (20, 40) being in contact with the air filter (24) includes a plurality of air holes to discharge the air separated from the fluid by the air filter (24) from the fluid path to the outside of the fluid path.

6. The warming device (100) according to claim 1, wherein a part of any one of the cover members (20, 40) being in contact with the air filter (24) includes a plurality of air holes to discharge the air separated from the fluid by the air filter (24) from the fluid path to the outside of the fluid path and wherein each of the plurality of air holes is formed therein with a check valve so that air is discharged from the fluid path and prevented from flowing into the fluid path from the outer of the warming device.

7. The warming device (100) according to claim 2, wherein the PCB type heater include at least one voltage electrode extended to the outer of the warming device (100).

8. The warming device (100) according to claim 7, wherein the at least one voltage electrode receives electric power from an external power supply and supplies electric power to the resistive pattern of the PCB type heater, or.
wherein the resistive pattern on the first side and second side of the PCB type heater is electrically insulated.

9. The warming device (100) according to claim 8, wherein the warming device is installed in a housing that is configured to receive the warming device therein and to connect the at least one voltage electrode and at least one temperature sensing electrode.

10. The warming device (100) according to claim 2, wherein the PCB type heater is connected to the at least one temperature sensing electrode and the at least one voltage electrode extended to the outer of the warming device, and includes at least one temperature sensor for measuring temperature of the fluid in the fluid path.

11. The warming device (100) according to claim 10, wherein the warming device (100) is installed in a housing that is configured to receive the warming device therein and to connect the warming device (100) to the at least one temperature sensing electrode.

12. The warming device (100) according to claim 1, wherein the barrier members (27) are formed on the first side and second side of the heater.

13. The warming device (100) according to claim 12:-
wherein the heater (50) comprises a connection hole for connecting the barrier members (27) formed on the first side and second side of the heater (50) to each other, or
further comprising a coating material formed on both sides of the heater (50), wherein the coating material has a melting point lower than a material forming the barrier members (27) so that the coating material is dissolved when the barrier members are formed on both sides of the heater (50), thereby resulting in forming the heater (50) and the barrier members (27) in a single body, or
further comprising a plurality of holes formed on an internal side of each of the cover members to receive ends of the barrier members (27) formed on the first side and the second side of the heater (50) when the cover members (20, 40) are combined together to form the fluid path surrounding the heater (50) several times.

14. The warming device (100) according to claim 3, further comprising a second barrier member formed on the first side and the second side of the heater (50), wherein the end of the barrier member formed on an inner side of each of the cover member is formed to be engaged with a corresponding shaped end of the second barrier member on the heater (50).

## Patentansprüche

1. Wärmevorrichtung (100), umfassend:
ein Heizgerät (50) mit einer ersten Seite und einer zweiten Seite;
einen ersten Verbindungsabschnitt (22), in den Fluid von einer externen Zuführungsvorrichtung fließt;
einen zweiten Verbindungsabschnitt (21), von dem erwärmtes Fluid abgegeben wird;
eine Vielzahl von Barriereelementen (27); und
ein Abdeckelementepaar (20, 40),
wobei die Wärmevorrichtung (50) derart ausgelegt ist, dass das Abdeckelementepaar (20, 40) jeweils auf gegenüberliegenden Seiten des Heizgeräts (50) angeordnet ist, wobei das Abdeckelementepaar (20, 40) das Heizgerät (50) und die Barriereelemente (27) zusammen kombiniert, und wobei ein Fluidweg, der das Heizgerät (50) mehrmals umgibt, zwischen dem Heizgerät (50) und den Barriereelementen (27) ausgebildet ist, so dass das vom ersten Verbindungsabschnitt (22) durch den Fluidweg fließende Fluid erwärmt wird und das erwärmte Fluid dem zweiten Verbindungsabschnitt (21) zugeführt wird, **dadurch gekennzeichnet, dass**: die Vielzahl von Barriereelementen auf jeder der Seiten des Heizgeräts angeordnet sind und die Wärmevorrichtung (100) ferner einen Luftfilter (24) umfasst, der zur Entfernung von Luft aus dem Fluid im Fluidweg angeordnet ist, wobei der Luftfilter (24) auf einer Innenseite eines beliebigen der Abdeckelemente (20, 40) angeordnet ist.

2. Wärmevorrichtung (100) nach Anspruch 1, wobei das Heizgerät (50) ein Leiterplatten-Heizgerät ist, das auf einem isolierenden Substrat gebildet ist, und das Leiterplatten-Heizgerät ein resistives Muster umfasst, das auf beiden Seiten des isolierenden Substrats ausgebildet ist.

3. Wärmevorrichtung (100) nach Anspruch 1, wobei die Barriereelemente (27) auf einer Innenseite jedes der Abdeckelemente (20, 40) ausgebildet sind.

4. Wärmevorrichtung (100) nach Anspruch 3, wobei die Abdeckelemente (20, 40) und die Barriereelemente (27) jeweils in einem einzelnen Körper durch Spritzgießen gebildet werden und/oder wobei ein Barriereelement der Barriereelemente (27) auf einem ersten Abdeckelement (20) der Abdeckelemente (20, 40) in einer ersten Richtung geneigt ist und ein Barriereelement der Barriereelemente (27) auf einem zweiten Abdeckelement (40) der Abdeckelemente (20, 40) in einer zweiten Richtung geneigt ist, so dass der Fluidweg in einer Schraubengewindeform ausgebildet sein kann.

5. Wärmevorrichtung (100) nach Anspruch 1,
wobei der Luftfilter (24) zwischen einer Innenseite eines beliebigen der Abdeckelemente (20, 40) und der Barriereelemente (27) angeordnet ist, oder
wobei der Luftfilter (24) aus einer hydrophoben Membran gebildet ist, um zu verhindern, dass das Fluid durch den Fluidweg fließt, oder
wobei ein Teil eines beliebigen der Abdeckelemente (20, 40), das mit dem Luftfilter (24) in Kontakt ist, eine Vielzahl von Luftlöchern zum Abgeben der Luft, die aus dem Fluid vom Luftfilter (24) abgetrennt wurde, vom Fluidweg zur Außenseite des Fluidwegs aufweist.

6. Wärmevorrichtung (100) nach Anspruch 1, wobei ein Teil eines beliebigen der Abdeckelemente (20, 40), das mit dem Luftfilter (24) in Kontakt ist, eine Vielzahl von Luftlöchern zum Abgeben der Luft, die aus dem Fluid vom Luftfilter (24) abgetrennt wurde, vom Fluidweg zur Außenseite des Fluidwegs aufweist, und wobei jedes der Vielzahl von Luftlöchern darin mit einem Rückschlagventil ausgebildet ist, so dass Luft aus dem Fluidweg abgegeben wird und daran gehindert wird, von der Außenseite der Wärmevorrichtung in den Fluidweg zu strömen.

7. Wärmevorrichtung (100) nach Anspruch 2, wobei das Leiterplatten-Heizgerät mindestens eine Spannungselektrode aufweist, die sich zur Außenseite der Wärmevorrichtung (100) erstreckt.

8. Wärmevorrichtung (100) nach Anspruch 7, wobei die mindestens eine Spannungselektrode elektrischen Strom von einer externen Stromversorgung erhält und dem resistiven Muster des Leiterplatten-Heizgeräts elektrischen Strom zuführt, oder
wobei das resistive Muster auf der ersten Seite und der zweiten Seiten des Leiterplatten-Heizgeräts elektrisch isoliert ist.

9. Wärmevorrichtung (100) nach Anspruch 8, wobei die Wärmevorrichtung in einem Gehäuse installiert ist, das zur Aufnahme der Wärmevorrichtung darin und zur Verbindung der mindestens einen Spannungselektrode und mindestens einer Temperaturmesselektrode ausgelegt ist.

10. Wärmevorrichtung (100) nach Anspruch 2, wobei das Leiterplatten-Heizgerät mit der mindestens einen Temperaturmesselektrode und der mindestens einen Spannungselektrode, die sich zur Außenseite der Wärmevorrichtung erstreckt, verbunden ist und mindestens einen Temperatursensor zur Messung der Temperatur des Fluids im Fluidweg aufweist.

11. Wärmevorrichtung (100) nach Anspruch 10, wobei die Wärmevorrichtung (100) im Gehäuse installiert ist, das zur Aufnahme der Wärmevorrichtung darin und zur Verbindung der Wärmevorrichtung (100) mit der mindestens einen Temperaturmesselektrode ausgelegt ist.

12. Wärmevorrichtung (100) nach Anspruch 1, wobei die Barriereelemente (27) auf der ersten Seite und der zweiten Seite des Heizgeräts ausgebildet sind.

13. Wärmevorrichtung (100) nach Anspruch 12:-
wobei das Heizgerät (50) ein Verbindungsloch zur Verbindung der Barriereelemente (27), die auf der ersten Seite und der zweiten Seite des Heizgeräts (50) ausgebildet sind, miteinander umfasst, oder
ferner umfassend ein Beschichtungsmaterial, das auf beiden Seiten des Heizgeräts (50) ausgebildet ist, wobei das Beschichtungsmaterial einen Schmelzpunkt aufweist, der niedriger als der eines Materials ist, das die Barriereelemente (27) bildet, so dass das Beschichtungsmaterial aufgelöst wird, wenn die Barriereelemente auf beiden Seiten des Heizgeräts (50) ausgebildet sind, was darin resultiert, dass das Heizgerät (50) und die Barriereelemente (27) in einem einzelnen Körper geformt werden, oder
ferner umfassend eine Vielzahl von Löchern, die auf einer Innenseite jedes der Abdeckelemente ausgebildet sind, um Enden der Barriereelemente (27), die auf der ersten Seite und der zweiten Seite des Heizgeräts (50) ausgebildet sind, aufzunehmen, wenn die Abdeckelemente (20, 40) zur Bildung des Fluidwegs, der das Heizgerät (50) mehrmals umgibt, kombiniert werden.

14. Wärmevorrichtung (100) nach Anspruch 3, ferner umfassend ein zweites Barriereelement, das auf der ersten Seite und der zweiten Seite des Heizgeräts (50) ausgebildet ist, wobei das Ende des Barriereelements, das auf einer Innenseite jedes der Abdeckelemente ausgebildet ist, so geformt ist, dass es mit einem entsprechend geformten Ende des zweiten Barriereelements auf dem Heizgerät (50) in Eingriff gebracht wird.

## Revendications

1. Dispositif de réchauffement (100) comprenant :
un élément chauffant (50) ayant un premier côté et un second côté ;
une première portion de raccordement (22) dans laquelle un fluide s'écoule depuis un dispositif d'alimentation externe ;
une seconde portion de raccordement (21) depuis laquelle un fluide réchauffé est évacué ;
une pluralité d'organes-barrières (27) ; et
une paire d'organes couvercles (20, 40),
dans lequel le dispositif de réchauffement (100) est configuré de manière à ce que les organes couvercles de la paire (20, 40) soient disposés chacun sur des côtés opposés de l'élément chauffant (50), la paire d'organes couvercles (20, 40) combine l'élément chauffant (50) et les organes-barrières (27) ensemble, et un chemin fluidique entourant l'élément chauffant (50) plusieurs fois est formé entre l'élément chauffant (50) et les organes-barrières (27), de sorte que le fluide s'écoulant à travers le chemin fluidique depuis la première portion de raccordement (22) soit réchauffé et que le fluide réchauffé soit fourni à la seconde portion de raccordement (21), **caractérisé en ce que** : les organes-barrières de la pluralité sont disposés sur chacun des côtés de l'élément chauffant, et le dispositif de réchauffement (100) comprend en outre un filtre à air (24) disposé dans le chemin fluidique pour éliminer l'air du fluide, dans lequel le filtre à air (24) est disposé sur un côté interne de l'un quelconque des organes couvercles (20, 40).

2. Dispositif de réchauffement (100) selon la revendication 1, dans lequel l'élément chauffant (50) est un élément chauffant de type PCB formé sur un substrat isolant, et l'élément chauffant de type PCB comprend un motif résistif formé sur les deux côtés du substrat isolant.

3. Dispositif de réchauffement (100) selon la revendication 1, dans lequel les organes-barrières (27) sont formés sur un côté intérieur de chacun des organes couvercles (20, 40).

4. Dispositif de réchauffement (100) selon la revendication 3, dans lequel les organes couvercles (20, 40) et les organes-barrières (27) sont formés chacun en un seul corps par un moulage par injection, respectivement, et/ou dans lequel un organe-barrière des organes-barrières (27) sur un premier organe couvercle (20) des organes couvercles (20, 40) est incliné dans une première direction et un organe-barrière des organes-barrières (27) sur un second organe couvercle (40) des organes couvercles (20, 40) est incliné dans une seconde direction de sorte que le chemin fluidique puisse être formé en une forme de filetage de vis.

5. Dispositif de réchauffement (100) selon la revendication 1,
dans lequel le filtre à air (24) est disposé entre un côté interne de l'un quelconque des organes couvercles (20, 40) et des organes-barrières (27), ou dans lequel le filtre à air (24) est composé d'une membrane hydrophobe pour empêcher le fluide de traverser le chemin fluidique ou
dans lequel une partie de l'un quelconque des organes couvercles (20, 40) en contact avec le filtre à air (24) comporte une pluralité de trous à air pour évacuer l'air séparé du fluide par le filtre à air (24) depuis le chemin fluidique vers l'extérieur du chemin fluidique.

6. Dispositif de réchauffement (100) selon la revendication 1, dans lequel une partie de l'un quelconque des organes couvercles (20, 40) en contact avec le filtre à air (24) comporte une pluralité de trous à air pour évacuer l'air séparé du fluide par le filtre à air (24) depuis le chemin fluidique vers l'extérieur du chemin fluidique et dans lequel à l'intérieur de chacun de la pluralité de trous à air est formé un clapet antiretour de façon à évacuer l'air du chemin fluidique et à l'empêcher de s'écouler dans le chemin fluidique depuis l'extérieur du dispositif de réchauffement.

7. Dispositif de réchauffement (100) selon la revendication 2, dans lequel l'élément chauffant de type PCB comporte au moins une électrode de tension étendue jusque l'extérieur du dispositif de réchauffement (100).

8. Dispositif de réchauffement (100) selon la revendication 7, dans lequel l'au moins une électrode de tension reçoit une alimentation électrique en provenance d'un bloc d'alimentation externe et fournit une alimentation électrique au motif résistif de l'élément chauffant de type PCB, ou
dans lequel le motif résistif sur le premier côté et le second côté de l'élément chauffant de type PCB est isolé électriquement.

9. Dispositif de réchauffement (100) selon la revendication 8, dans lequel le dispositif de réchauffement est installé dans un boîtier qui est configuré pour y recevoir le dispositif de réchauffement et pour raccorder l'au moins une électrode de tension et au moins une électrode de détection de température.

10. Dispositif de réchauffement (100) selon la revendication 2, dans lequel l'élément chauffant de type PCB est raccordé à l'au moins une électrode de détection de température et l'au moins une électrode de tension étendue jusque l'extérieur du dispositif de réchauffement, et comporte au moins un capteur de température pour mesurer une température du fluide dans le chemin fluidique.

11. Dispositif de réchauffement (100) selon la revendication 10, dans lequel le dispositif de réchauffement (100) est installé dans un boîtier qui est configuré pour y recevoir le dispositif de réchauffement et pour raccorder le dispositif de réchauffement (100) à l'au moins une électrode de détection de température.

12. Dispositif de réchauffement (100) selon la revendication 1, dans lequel les organes-barrières (27) sont formés sur le premier côté et le second côté de l'élément chauffant.

13. Dispositif de réchauffement (100) selon la revendication 12 :
dans lequel l'élément chauffant (50) comprend un trou de raccordement pour raccorder les organes-barrières (27) formés sur le premier côté et le second côté de l'élément chauffant (50) l'un à l'autre, ou
comprenant en outre un matériau de revêtement formé sur les deux côtés de l'élément chauffant (50), dans lequel le matériau de revêtement a un point de fusion inférieur à un matériau formant les organes-barrières (27) de sorte que le matériau de revêtement soit dissous lorsque les organes-barrières sont formés sur les deux côtés de l'élément chauffant (50), aboutissant ainsi à la formation de l'élément chauffant (50) et des organes-barrières (27) en un seul corps, ou
comprenant en outre une pluralité de trous formés sur un côté interne de chacun des organes couvercles pour recevoir des extrémités des organes-barrières (27) formés sur le premier côté et le second côté de l'élément chauffant (50) lorsque les organes couvercles (20, 40) sont combinés ensemble pour former le chemin fluidique entourant l'élément chauffant (50) plusieurs fois.

14. Dispositif de réchauffement (100) selon la revendication 3, comprenant en outre un second organe-barrière formé sur le premier côté et le second côté de l'élément chauffant (50), dans lequel l'extrémité de l'organe-barrière formé sur un côté intérieur de chacun des organes couvercles est formée pour être enclenchée avec une extrémité de forme correspondante du second organe-barrière sur l'élément chauffant (50).
